(19) Europäisches Patentamt
European Patent Office
Office européen des brevets

(11) Publication number: 0 244 948 A2

# (12) EUROPEAN PATENT APPLICATION

(21) Application number: 87302915.1

(22) Date of filing: 03.04.87

(51) Int. Cl.4: **C07D 249/12** , C07D 487/04 , A01N 43/653 , A01N 43/90 , //(C07D487/04,249:00,239:00),(-C07D487/04,249:00,235:00)

(30) Priority: **30.04.86 GB 8610532**
**03.10.86 GB 8623846**

(43) Date of publication of application: **11.11.87 Bulletin 87/46**

(84) Designated Contracting States: **AT BE CH DE ES FR GB GR IT LI LU NL SE**

(71) Applicant: **SCHERING AGROCHEMICALS LIMITED**

**Hauxton, Cambridge CB2 5HU(GB)**

(72) Inventor: **Green, David Eric**
**5 Emsons Close**
**Linton Cambridge(GB)**

(74) Representative: **Wells, Norman David et al**
**Schering Agrochemicals Limited Industrial Property Department Chesterford Park Research Station**
**Saffron Walden Essex CB10 1XL(GB)**

## (54) Triazolopyrimidine herbicides.

(57) The invention provides herbicidal triazolesulphonamides, processes for their preparation and compositions containing them, the compounds being of the formula:

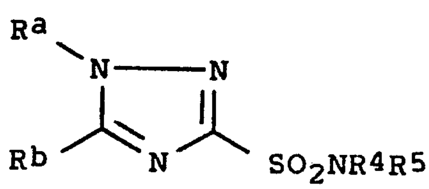

(I)

and the salts thereof, where $R^a$ represents hydrogen and $R^b$ represents a group $R^3CONHCR^2=CR^1$-, or $R^a$ and $R^b$ together represent a chain of formula
$-C(R^3)=N-C(R^2)=C(R^1)-$or
$-C(=Y)-N(R^6)-C(R^2)=C(R^1)-$, where $R^1$, $R^2$ and $R^3$, which may be the same or different, each represent hydrogen, hydroxy, mercapto, halo, cyano, amino, alkylamino, dialkylamino, or substituted or unsubstituted alkyl, aryl, aralkyl, alkoxy or alkylthio, Y is oxygen or sulphur, and $R^6$ is hydrogen or a substituted or unsubstituted alkyl or aryl group; or $R^1$ and $R^2$ together represent $-CH=CH-CH=CH-$or $-(CH_2)_n-$where n is 3, 4 or 5; $R^4$ represents substituted phenyl or a substituted or unsubstituted aralkyl or heteroaryl group; and $R^5$ represents hydrogen, acyl, carboxy, lkoxycarbonyl, alkylsulphonyl, or a substituted or unsubstituted alkyl, alkenyl, alkynyl, aryl or aralkyl group, or a group of the formula:

EP 0 244 948 A2

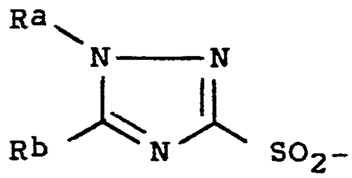

(A)

where $R^a$ and $R^b$ are as defined hereinbefore.

# Triazolopyrimidine herbicides

This invention concerns herbicidal triazole sulphonamides, processes for their preparation, and compositions containing them.

In one aspect, the invention provides the triazole sulphonamides of the formula:

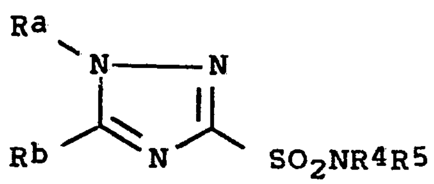

(I)

and the salts thereof, where $R^a$ represents hydrogen and $R^b$ represents a group $R^3CONHCR^2=CR^1$-, or $R^a$ and $R^b$ together represent a chain of formula
$-C(R^3)=N-C(R^2)=C(R^1)$-or
$-C(=Y)-N(R^6)-C(R^2)=C(R^1)-$, where $R^1$, $R^2$ and $R^3$, which may be the same or different, each represent hydrogen, hydroxy, mercapto, halo, cyano, amino, alkylamino, dialkylamino, or substituted or unsubstituted alkyl, aryl, aralkyl, alkoxy or alkylthio, Y is oxygen or sulphur, and $R^6$ is hydrogen or a substituted or unsubstituted alkyl or aryl group; or $R^1$ and $R^2$ together represent $-CH=CH-CH=CH$-or $-(CH_2)_n$-where n is 3, 4 or 5; $R^4$ represents substituted phenyl or a substituted or unsubstituted aralkyl or heteroaryl group; and $R^5$ represents hydrogen, acyl, carboxy, alkoxycarbonyl, alkylsulphonyl, or a substituted or unsubstituted alkyl, alkenyl, alkynyl, aryl or aralkyl group, or a group of the formula:

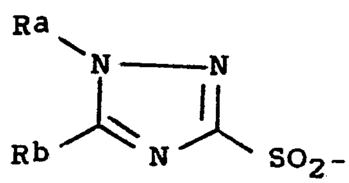

(A)

where $R^a$ and $R^b$ are as defined hereinbefore.

$R^a$ and $R^b$ preferably together represent a chain of formula $-C(R^3)=N-C(R^2)=C(R^1)-$.

When $R^1$, $R^2$ or $R^3$ represents halo, it is preferably chloro or bromo.

When $R^1$, $R^2$, $R^3$, $R^5$ or $R^6$ represents or contains an alkyl group, that group is preferably of 1 to 6 carbon atoms, especially of 1 to 4 carbon atoms. Specific preferred unsubstituted alkyl-containing groups include methyl, ethyl, n-propyl, isopropyl, n-butyl, t-butyl, methoxy, ethoxy, n-propoxy, methylamino, dimethylamino, methylthio and ethylthio. The alkyl group may if desired be substituted, for example by one or more halogen atoms, eg fluorine, chlorine or bromine, or by alkoxy groups of 1 to 4 carbon atoms, eg methoxy or ethoxy. Specific preferred substituted alkyl-containing groups include chloromethyl, bromomethyl, dichloromethyl, trifluoromethyl, methoxyethyl and ethoxyethyl.

When $R^1$, $R^2$, $R^3$, $R^5$ or $R^6$ represents an aryl or aralkyl group, it is preferably a phenyl group or an aralkyl group of 7 to 10 carbon atoms, especially benzyl, substituted for example by one or more halogen atoms, eg fluorine, chlorine or bromine, nitro groups, cyano groups, or alkyl or alkoxy groups of 1 to 4 carbon atoms, eg methyl, ethyl, methoxy or ethoxy groups.

$R^1$, $R^2$ and $R^3$ independently preferably represent hydrogen, methyl, ethyl, phenyl, trifluoromethyl, chloro or bromo.

It will be appreciated that when $R^3$ represents hydroxy or mercapto, the compounds may exist in either the keto or enol forms or as an equilibrium mixture of the two.

When $R^4$ or R5 represents an aralkyl group, it is preferably benzyl, which may be substituted by one or more alkyl or alkoxy groups of 1 to 4 carbon atoms, halogen atoms or nitro groups.

When $R^4$ represents a heteroaryl group, it is preferably a 5-or 6-membered heterocycle which contains one or more nitrogen, oxygen or sulphur atoms. Preferred such groups include pyridyl, pyrrolyl, furyl and thienyl.

$R^4$ preferably represents a phenyl group substituted by one or more halogen (eg chlorine, bromine or fluorine) atoms, alkyl groups of 1 to 4 carbon atoms (eg methyl or ethyl), alkoxy or alkylthio groups of 1 to 4 carbon atoms (eg methoxy, ethoxy or methylthio) trifluoromethyl groups, alkoxycarbonyl groups of 2 to 5 carbon atoms (eg methoxycarbonyl or ethoxycarbonyl) or nitro groups.

When $R^5$ represents an alkyl group, it is preferably methyl or ethyl. When, however it represents an alkenyl or alkynyl group, that group is preferably unsubstituted, and is preferably of 2 to 6 carbon atoms, for example vinyl, allyl or propargyl. Preferred acyl, alkoxycarbonyl and alkylsulphonyl groups which $R^5$ may represent are those in which the alkyl moiety is of 1 to 4 carbon atoms, including formyl, acetyl, propanoyl, methoxycarbonyl, ethoxycarbonyl and methylsulphonyl.

$R^5$ preferably represents hydrogen.

Preferred salts of the compounds of formula I are alkali-metal salts, eg sodium or potassium salts, ammonium salts, trialkylammonium salts, and salts with heterocyclic bases, eg the pyridinium salts.

In a particularly preferred group of compounds of formula I, $R^a$ and $R^b$ together represent a chain of formula $-C(R^3)=N-C(R^2)=C(R^1)-$, $R^1$, $R^2$ and $R^3$ each represent hydrogen, methyl, ethyl, phenyl, trifluoromethyl or chloro, $R^4$ is a phenyl group substituted by one or more halogen atoms, alkyl, alkoxy or alkylthio groups of 1 to 4 carbon atoms, trifluoromethyl groups, alkoxycarbonyl groups of 2 to 5 carbon atoms, or nitro groups, and $R^5$ is hydrogen.

Especially preferred compounds according to the invention are those of the Examples provided hereinafter. Specific mention may be made, however, of N-(2,6-difluorophenyl)-5,7-dimethyl[1,2,4]triazolo-[1,5-c]pyrimidine-2-sulphonamide and 5-(2-acetamido-1-propenyl)-N-(2,6-difluorophenyl)-1H-1,2,4-triazole-3-sulphonamide.

In another aspect, the invention provides a process for the preparation of a triazole sulphonamide of formula I where $R^a$ and $R^b$ together represent a chain of formula $-C(R^3)=N-C(R^2)=C(R^1)-$or $-C(=Y)-N(R^6)-C-(R^2)=C(R^1)-$, where $R^1$, $R^2$, $R^3$, $R^6$ and Y are as defined hereinbefore, in which a sulphonyl halide of the formula:

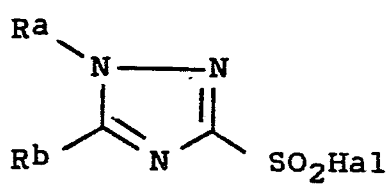

(II)

where $R^a$ and $R^b$ are as defined hereinbefore, and Hal represents halogen, is reacted in the presence of a base with an amine of the formula $R^4R^5NH$ where $R^4$ and $R^5$ are as defined hereinbefore to give the desired compound.

The reaction is conveniently effected at a temperature of from 0°C to 100°C, for example 0 to 50°C, especially at room temperature. It is preferably effected in a suitable solvent medium, especially pyridine, which may also serve as the base.

The reaction may be catalysed by a stronger organic base, particularly a tertiary organic base such as 4-dimethylaminopyridine.

The compounds of formula II may themselves be prepared by reacting a compound of the formula:

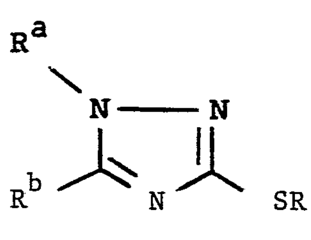

(III)

where $R^a$ and $R^b$ together represent a chain of formula $-C(R^3)=N-C(R^2)=C(R^1)-$or $-C(=Y)-N(R^6)-C(R^2)=C-(R^1)-$where $R^1$, $R^2$, $R^3$, $R^6$ and Y are as defined hereinbefore, and R represents hydrogen, alkyl, aryl or aralkyl, with the appropriate halogen or sulphuryl halide in a suitable solvent medium, to give the desired compound.

The reaction is desirably effected with cooling to less than ambient temperature, eg to a temperature of -10°C to 5°C.

Where a halogen is used, it is conveniently passed into a solution or suspension of the appropriate starting material in an aqueous medium, eg aqueous acetic acid or hydrochloric acid.

Where a sulphuryl halide is employed, the reaction is preferably effected in the presence of wet silica gel, and in a suitable solvent medium, eg dichloromethane.

The compounds of formula I where $R^a$ represents hydrogen and $R^b$ represents $R^3CONHCR^2=CR^1$-may be prepared by a process which comprises subjecting the corresponding compounds of formula I where $R^a$ and $R^b$together represent $-C(R^3)=N-C(R^2)=C(R^1)$-to the action of a base under mild conditions to give the desired compound.

The base is desirably an inorganic base, especially an alkali-metal hydroxide, eg sodium or potassium hydroxide, and is desirably employed in aqueous solution.

The reaction is desirably effected at a temperature of from 0°C to 50°C, particularly at room temperature. At higher temperatures, or under more severe conditions, the reaction proceeds a stage further as described hereinafter.

The compounds of formula I where $R^a$ and $R^b$together represent a chain $-C(R^3)=N-C(R^2)=C(R^1)$-where $R^3$ is other than hydroxy, mercapto or amino may alternatively be prepared by a process in which a compound of the formula:

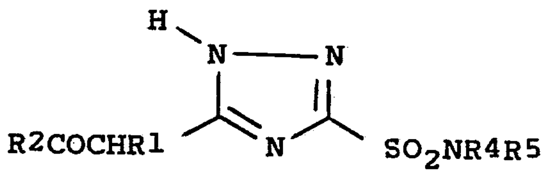

(IV)

where $R^1$, $R^2$, $R^4$, and $R^5$ are as defined hereinbefore is reacted with an amide of formula $R^3CONH_2$ where $R^3$ is as defined herinbefore to give the desired compound.

The reaction is conveniently carried out by heating a mixture of the reactants, eg to 50°C to 200°C.

The compounds of formula I in which $R^a$ and $R^b$together represent a chain $-C(R^3)=N-C(R^2)=C(R^1)$-where $R^3$ is hydroxy, mercapto or amino may be prepared by reacting a compound of formula IV as defined hereinbefore respectively with urea, thiourea or guanidine, to give the desired compound.

The reaction is conveniently carried out by heating a mixture of the reactants, eg to 50°C to 200°C.

The compounds of formula IV may in turn be prepared by a process in which a compound of formula I in which $R^a$represents hydrogen and $R^b$ represents a group $R^3CONHCR^2=CR^1$-is subjected to the action of a suitable base under suitable conditions to give the desired compound.

The base employed is conveniently an alkali-metal base, eg an alkali-metal hydroxide, and the reaction is desirably effected in a suitable solvent medium, eg water, and with heating to a temperature of from 60°C to 100°C.

The compounds of formula III in which $R^a$ and $R^b$together represent a chain of formula $-C(=Y)-N(R^6)-C(R^2)=C(R^1)$-, where Y, $R^1$, $R^2$ and $R^6$ are as defined herinbefore are themselves novel compounds which may be prepared from the corresponding compounds in which $R^a$ is hydrogen and $R^b$ is a group $R^2COCHR^1$-by reaction thereof with an isocyanate or isothiocyanate of formula $R^6NCY$ where $R^6$ and Y are as defined hereinbefore. In turn these staring materials may be made by.reacting thiosemicarbazide with a compound of the formula:

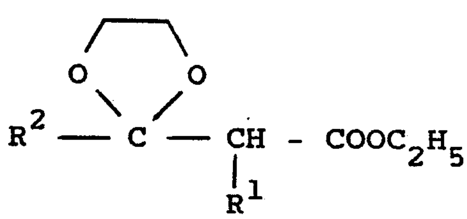

(V)

where $R^1$ and $R^2$ are as defined hereinbefore, reacting the product, if desired, with a compound of formula RHal where R is as defined hereinbefore and removing the protecting group by means of acid.

Salts of the compounds of formula I may be prepared by reaction thereof with a suitable base containing the desired cation, eg the appropriate trialkylamine.

The comounds of formula I may also be interconverted by methods known per se. For example, the compounds of formula I in which $R^5$ is other than hydrogen may be prepared from the corresponding compounds of formula I where $R^5$ is hydrogen by reaction thereof in the presence of a strong base, for example sodium hydride, with a halide of formula $R^5Hal$.

Carboxy groups present in the compounds of formula I may be esterified, ester groups present may be hydrolysed to give the corresponding acids, and cyano or ester groups may be converted to amido or substituted amido groups. Such transformations, as well as many others, may be effected by techniques well-known to those skilled in the art, and various examples of such transformations are provided hereinafter.

Particular mention may be made of the conversion of compounds of formula I in which $R^2$ or $R^3$ is hydroxy into the corresponding compounds in which $R^3$ is chloro by reaction of the former with phosphorus oxychloride, and the subsequent reaction of the chloro compounds in the presence of a base with an appropriate alcohol, alkanethiol, amine or cyanide to give the corresponding compounds of formula I in which $R^3$ respectively represents alkoxy, alkylthio, amino or cyano.

The compounds of formula I are herbicidally-active against a wide range of broad-leaved and grassy weeds, but are comparatively safe to certain crop species. They may thus be of use as selective herbicides, particularly in the control of a range of weeds in cereals or other crops, eg wheat, barley, maize, soya beans, oilseed rape, cotton or sugar beet.

In another aspect, the invention provides a herbicidal composition which comprises one or more compounds of the invention in association with a suitable carrier and/or surface active agent.

The compositions usually contain from 0.01 to 99% by weight of the present compounds, and are normally produced initially as concentrates containing from 0.5 to 99%, preferably from 0.5 to 85%, and especially from 10 to 50% by weight thereof. Such concentrates are diluted if necessary before application to the locus to be treated such that the active ingredient comprises from 0.01 to 5% by weight of the formulation applied.

The carrier may be water, in which case an organic solvent may also be present, though this is not usually employed. A flowable suspension concentrate may be formed by grinding the compound with water, a wetting agent and a suspending agent, e.g. xanthan gum.

The carrier may alternatively be a water immiscible organic solvent, e.g. a hydrocarbon which boils within the range 130-270°C, e.g. xylene, in which the compound is dissolved or suspended. An emulsifiable concentrate containing a water immiscible solvent may be formed with a surface active agent so that the concentrate acts as a self-emulsifiable oil on admixture with water.

The carrier may alternatively be a water-miscible organic solvent e.g. 2-methoxy ethanol, methanol, propylene glycol, diethylene glycol, diethylene glycol monoethyl ether, methylformamide or dimethylformamide.

The carrier may alternatively be a solid, which may be finely divided or granular. Examples of suitable solids are limestone, clays, sand, mica, chalk, attapulgite, diatomite, perlite, sepiolite, silicas, silicates, lignosulphonates and solid fertilizers. The carrier can be of natural or synthetic origin or can be modified natural material.

Wettable powders soluble or dispersible in water may be formed by admixing the compound in particulate form with a particulate carrier or spraying molten compound on to the particulate carrier, admixing a wetting agent and a dispersing agent and finely grinding the whole powder mixture.

An aerosol composition may be formed by admixing the compound with a propellant, e.g. a polyhalogenated alkane such as dichlorofluoromethane, and suitably also with a solvent.

The term 'surface active agent' is used in the broad sense to include materials variously called emulsifying agents, dispersing agents and wetting agents. Such agents are well known in the art.

The surface active aqents used may comprise anionic surface active agents, for example mono-or diesters of phosphoric acid with a fatty alcohol ethoxylate, or salts of such esters, fatty alcohol sulphates such as sodium dodecyl sulphate, ethoxylated fatty alcohol sulphates, ethoxylated alkylphenol sulphates, lignin sulphates, petroleum sulphonates, alkylaryl sulphonates such as alkyl-benzene sulphonates or lower alkylnaphthalene sulphonates, salts of sulphonated naphthaleneformaldehyde condensates, salts of sulphonated phenolformaldehyde condensates, or more complex sulphonates such as the amide sulphonates, e.g. the sulphonated condensation product of oleic acid and N-methyl taurine or the dialkyl sulphosuccinates e.g. the sodium sulphonate of dioctyl succinate.

The surface active agents may also comprise non-ionic agents, for example condensation products or fatty acid esters, fatty alcohols, fatty acid amides or alkyl-substituted phenols with ethylene oxide, fatty esters of polyhydric alcohol ethers e.g. sorbitan fatty acid esters, condensation products of such esters with ethylene oxide e.g. polyoxyethylene sorbitan fatty acid esters, block copolymers of ethylene oxide and propylene oxide, acetylenic glycols such as 2,4,7,9-tetramethyl-5-decyn-4,7-diol, or ethoxylated acetylenic glycols.

The surface active agents may also comprise cationic agents, for example alkyl-and/or aryl-substituted quaternary ammonium compounds such as cetyl trimethylammonium bromide, or ethoxylated tertiary fatty amines.

Preferred surface active agents include ethoxylated fatty alcohol sulphates, lignin sulphonates, alkyl-aryl sulphonates, salts of sulphonated naphthaleneformaldehyde condensates, salts of sulphonated phenolformaldehyde condensates, sodium oleoyl N-methyltauride, dialkyl sulphosuccinates, alkyl phenol ethoxylates, and fatty alkyl ethoxylates.

The present active compounds, especially those of the Examples provided hereinafter, and particularly N-(2,6-difluorophenyl)-5,7-dimethyl[1,2,4]triazolo[1,5-c]pyrimidine-2-sulphonamide and 5-(2-acetamido-1-propenyl)-N-(2,6-difluoropheny1)-1H-1,2,4-triazole-3-sulphonamide, may be admixed with another pesticide, eg a herbicide, fungicide or insecticide, or a plant growth regulator, particularly another herbicide. Suitable further herbicides include trietazine, linuron, MCPA, dichlorprop, isoxaben, diflufenican, metolachlor, fluometuron, oxyfluorfen, fomesafen, bentazone, prometryne, norflurazon, chlomazone, EPTC, imazaquin, and especially isoproturon, methabenzthiazuron, trifluralin, ioxynil, bromoxynil, benazolin, mecoprop, fluroxypyr, alachlor, acifluorfen, lactofen, metribuzin and pendimethalin.

The present compounds may be applied to plants, the soil, land or aquatic areas, and particularly to a locus at which a crop is growing or is about to grow. The compounds are active both pre-and post-emergence.

The invention is illustrated by the following Examples, in which Me = methyl, Et = ethyl and Ph = phenyl.

Preparative Example A

N-(2-chlorophenyl)-5,7-dimethyl[1,2,4]triazolo[1,5-c]pyrimidine-2-sulphonamide (Compound AI)

(a) 5,7-dimethyl[1,2,4]triazolo[1,5-c]pyrimidine-2-sulphonyl chloride

Chlorine gas was passed into a suspension of 5,7-dimethyl[1,2,4]triazolo[1,5-c]pyrimidine-2-thiol (5.4g) in water (50ml) at 5-10°C with vigorous stirring for 30 minutes. The solid was filtered off and dissolved in dichloromethane (80ml). The organic solution was washed with water (10ml), dried over magnesium sulphate and concentrated in vacuo to give a yellow gummy solid, 2.7g, shown to be the desired product by spectroscopy.

(b) N-(2-chlorophenyl)-5,7-dimethyl[1,2,4]triazolo[1,5-c]pyrimidine-2-sulphonamide

A solution of the sulphonyl chloride (2.7g) in dichloromethane (10ml) was slowly added at 10-15°C to a stirred solution of 2-chloroaniline (1.54g) in pyridine (20 ml). The temperature was maintained at below 15°C. After the addition was complete, the mixture was allowed to return to ambient temperature and was stirred overnight. It was then concentrated in vacuo and 5% hydrochloric acid (15ml) and ether (10ml) were added to the residue. The resulting solid was filtered and flash chromatographed on silica using petroleum ether (bp 60-80°C)/ethyl acetate 1:1 as eluent. The desired sulphonamide was obtained as a cream solid, mp 190-192°C, in a yield of 1g.

Examples A2-A49

The following compounds of formula I where $R^5$ represents hydrogen were prepared by methods analogous to that of Example A:

| No. | R1 | R2 | R3 | R4 (Ph subst:) | m.pt (°C) |
|---|---|---|---|---|---|
| A2 | H | Me | Me | 2,6-diCl | 279-282 |
| A3 | H | Me | Me | 2-Cl,6-Me | 238-239 |

| No. | R1 | R2 | R3 | R4 (Ph subst:) | m.pt (°C) |
|---|---|---|---|---|---|
| A4 | H | Me | Me | 2,6-diF | 229-231 |
| A5 | H | Me | Me | 2-$NO_2$ | 189-192 |
| A6 | H | Me | Me | 2-$CF_3$ | 193-195 |
| A7 | H | Me | Me | 2-COOMe | 180-182 |
| A8 | H | Me | Me | 2,6-diCl,3-Me | 242-244 |
| A9 | H | Me | Me | 2,3-diCl | 176-178 |
| A10 | Me | Me | Me | 2,6-diCl | 241-243 |
| A11 | Me | Me | Me | 2-Cl-6-Me | 212-214 |
| A12 | Me | Me | Me | 2,6-diCl,3-Me | 232-234 |
| A13 | Cl | Me | Me | 2,6-diF | 214-216 |
| A14 | H | Me | Me | 2-Cl,6-F | 253-255 |
| A15 | H | Me | Me | 2-Me,6-$NO_2$ | 248-250 |
| A16 | Cl | Me | H | 2,6-diF | 235-238 |
| A17 | H | Me | Ph | 2,6-diF | 266-269 |
| A18 | H | Me | Me | 2,3,5,6-tetraF | 236-239 |
| A19 | H | Ph | Me | 2,6-diF | 269-275 |
| A20 | H | Ph | Me | 2-Cl,6-Me | 272-275 |
| A21 | H | Me | Ph | 2-Cl,6-Me | 275-276 |
| A22 | H | $CF_3$ | Me | 2,6-diF | 165-167 |
| A23 | Me | Me | Me | 2,6-diF | 220-223 |
| A24 | H | Me | Me | 2-Cl,6-SMe | 233-235 |
| A25 | H | Et | Me | 2,6-diF | 190-192 |
| A26 | H | Me | OH | 2,6-diF | >350 |
| A27 | H | Me | Me | 2-Me,6-COOMe | 195-197 |

| No. | R1 | R2 | R3 | R4 (Ph subst:) | m.pt (°C) |
|---|---|---|---|---|---|
| A28 | H | Me | Me | 2,6-diBr | 276-278 |
| A29 | H | H | Me | 2,6-diF | 203-205 |
| A30 | Br | Me | H | 2,6-diF | 219-221 |
| A31 | H | Me | Me | 2,3-diMe,6-$NO_2$ | 177-179 |
| A32 | H | H | Me | 2,6-diCl | 222-224 |
| A33 | H | Me | Me | 2-F,6-COOMe | 213-215 |
| A34 | H | H | Me | 2-Cl,6-Me | 218-219 |
| A35 | H | Me | Me | 2,5-diMe,6-COOMe | 207-209 |
| A36 | H | Me | H | 2,6-diF | 202-204 |
| A37 | H | Me | Me | 2-F,6-COOEt | 160-163 |
| A38 | H | Me | Me | 2-$OCHF_2$ | 160-162 |
| A39 | H | Me | Me | 2,3-diCl-6-$NO_2$ | 229-234 |
| A40 | H | Me | Me | 2-OMe | 178-180 |
| A41 | H | Me | Me | 2-CN | 235-236 |
| A42 | H | Me | Me | 3,5-diCl | 267-269 |
| A43 | H | Me | Me | 2-$SO_2NH_2$ | 208-210 |
| A44 | H | Me | Me | 2,6-diMe | 235-237 |
| A45 | H | Me | Me | 2,5-diCOOMe | 176-179 |
| A46 | H | Me | Me | 2,5-diCl | 198-201 |

The following compound of formula I was also prepared by a method analogous to that of Example A:

| No. | R1 | R2 | R3 | R4 (Ph subst:) | R5 | m.pt (°C) |
|---|---|---|---|---|---|---|
| A47 | H | Me | Me | 2,6-diF | Me | 168-170 |

The following compound of formula I where $R^4$ represents 2-pyridyl was also prepared by a method analogous to that of Example A:

| No. | R1 | R2 | R3 | R5 | m.pt (°C) |
|---|---|---|---|---|---|
| A48 | H | Me | Me | H | 246-247 |

Examples A49-A52

The following compounds of formula I where $R^5$ represents a group of formula A where $R^1$, $R^2$ and $R^3$ are the same as the corresponding groups in the remainder of the molecule were prepared by methods analogous to those of Example A:

| No. | R1 | R2 | R3 | R4 (Ph subst:) | m.pt (°C) |
|---|---|---|---|---|---|
| A49 | H | Me | Me | 2,6-diCl,3-Me | 278-282 |
| A50 | H | Me | Me | 2-Me,6-$NO_2$ | 244-246 |
| A51 | H | Me | Me | 2-F,6-COOMe | 255-257 |
| A52 | H | Me | Me | 2-F,6-COOEt | 259-261 |

Preparative Example B

5-(2-Acetamido-1-propenyl)-N-(2,6-difluorophenyl)-1H-1,2,4-triazole-3-sulphonamide (Compound B1)

A solution of the product of Example 4 (2.4g) in 5% aqueous sodium hydroxide solution (20ml) was stirred at room temperature for 1.5 hours and was left to stand for a further 18 hours. The solution was then acidified to pH=2 with concentrated hydrochloric acid to precipitate a solid. This was filtered off, washed with water (2 $\times$ 10ml) and ether (2 $\times$ l0ml) to give 2.1g of the desired product as a white solid, mp 226-228°C.

Examples B2-B7

The following compounds of formula I where $R^5$ and $R^a$ are hydrogen and $R^b$ represents a group $R^3CONHCR^2=CR^1$-were prepared by methods analogous to those of Example B.

| No. | R1 | R2 | R3 | R4 (Ph subst:) | m.pt (°C) |
|---|---|---|---|---|---|
| B2 | H | Me | Me | 2-Cl,6-Me | 234-236 |
| B3 | H | $CF_3$ | Me | 2,6-diF | 141-145 |
| B4 | H | Me | Me | 2-Cl | 162-164 |
| B5 | H | Me | Me | 2,6-diCl,3-Me | 234-235 |
| B6 | H | Me | Me | 2,6-diCl | 282-283 |
| B7 | H | Me | Me | 2-Me,6-$NO_2$ | 242-245 |

Preparative Example C

N-(2,6-difluorophenyl)-5,6-dihydro-7-methyl-5-oxo[1,2,4]triazolo[1,5-c]pyrimidine-2-sulphonamide

An aqueous solution of the product of Example 4 (15g) in 5% sodium hydroxide (25ml) was heated at reflux for 4 hours. The solution was then cooled, and acidified to pH = 4 with concentrated hydrochloric acid to precipitate a cream solid. This was filtered off and washed with water and ether to give 11g of N-(2,6-difluorophenyl)-5-(2-oxopropyl)-1H-1,2,4-triazole-3-sulphonamide, mp 182-184°C.

An intimate mixture of the produced sulphonamide (2g) and urea 10g, were stirred at 150°C for 3 hours, after which the mixture was cooled to 70°C and water (40ml) was added. The solution was acidified to pH = 1 by the addition of concentrated hydrochloric acid to precipitate a solid which was removed by filtration. The solid was extracted with boiling acetonitrile, filtered hot and left to cool, which gave a solid precipitate. This was filtered off to give 0.48g of the desired product as a white solid, mp >350°C which was identical to the product of Example 26.

Preparative Example D

(a) 5-(2-Methyl-1,3-dioxolan-2-ylmethyl)-1H-1,2,4-triazole-3-thiol

Ethyl (2-methyl-1,3-dioxolan-2-ylmethyl)acetate (43.5g) was added dropwise to a suspension of thiosemicarbazide (22.8g) in sodium ethoxide solution (6.04g sodium in 500ml absolute alcohol) at room temperature. The suspension was heated at reflux for 18 hours. After cooling, the solid was removed by filtration, and the filtrate was concentrated in vacuo. The oily residue was dissolved in water (60ml), acidified to pH = 5 with acetic acid, and concentrated in vacuo. Trituration of the residue with propan-2-ol (50ml) and filtration gave 11.6g of the desired product, mp 168-173°C, as a white solid.

(b) 3-Benzylthio-5-(2-methyl-1,3-dioxolan-2-ylmethyl)-1H-1,2,4-triazole

Benzyl chloride (8.07g) was added dropwise at room temperature to a solution of the product of stage (a) (11.6g) in sodium ethoxide solution (1.4g sodium in 100ml absolute alcohol) and the mixture was stirred for 18 hours. The precipitated solid was removed by filtration, and the filtrate was concentrated in vacuo. The resulting oil was partitioned between water (60ml) and petroleum ether (bp 60-80) (50ml), and the solid was removed by filtration. This gave 13.9g of the desired product, mp 96-98°C, as a white solid.

(c) 1-(5-Benzylthio-1H-1,2,4-triazol-3-yl)propan-2-one

A suspension of the product of stage (b) (9.9g) in 1% hydrochloric acid (110ml) was heated at reflux for 30 minutes. The mixture was then cooled, and the solid removed by filtration. This solid was identified as the desired product, 7.6g, mp 125°C-127°C.

Preparative Example E

N-acetyl-N-(2,6-difluorophenyl)-5,7-dimethyl[1,2,4]triazolo[1,5-c]pyrimidine-2-sulphonamide

The product of Example A4 (2g) was heated with acetic anhydride (12ml) at reflux for 2 hours, after which the reaction mixture was allowed to cool. The precipitated solid was removed by filtration to give 1.2g of the desired product, mp 171-173°C.

Preparative Example F

Ethyl ch;oroformate (1.3g) was added dropwise to a suspension of the product of Example A4 (2.5g) in pyridine (13ml) at 22°C, and the mixture was stirred at room temperature for 2 days. After filtering off the remaining solid, the filtrate was acidified with hydrochloric acid, and the resulting solid was removed by filtration, washed with dilute ammonia (20ml) and recrystallised from ethyl acetate to give 1.9g of the desired product, mp 155-157°C.

FORMULATION EXAMPLES

A 10% suspension concentrate formulation was prepared from the following ingredients:

| Ingredient | g/l |
|---|---|
| Compound of Ex A4 | 100 |
| Pluronic P75 | 30 |
| Polyfon H | 20 |
| Propylene glycol | 105 |
| Anti-foam 1520 | 0.5 |
| Veegum R | 7.5 |
| Kelzan | 1.0 |
| Formaldehyde (40%) | 1.0 |
| Water | to 1 litre |

Corresponding formulations containing 5, 10, 25, 50, 200 and 500 g/l of the compounds of Examples A1-A53, B1-B7, C, D and E were also prepared.

HERBICIDAL EXAMPLE A (Pre-Emergence)

Seeds of the weed species listed below were sown in anodised aluminium pans 19 cm long x 9.5 cm wide x 6 cm deep, containing sterilized sandy loam. They were watered and then sprayed with the compounds of the Examples listed below formulated as a solution/suspension in 1:1 by volume of acetone and the wetting agent polyoxyethylene (20 mols) monolaurate solution (2 g per litre).

The concentration of each test compound and volume of application were calculated to give the desired rate of application of the compound in 450 litres per hectare. After 3 to 4 weeks growth in the controlled environment room (20°C; 75-95% relative humidity; 14 hours per day artificial illumination) the plants were visually assessed for any herbicidal response.

All differences from an untreated control were scored accordingly to an index where 0 = no effect, 1 = 1-24% effect, 2 = 25-69% effect, 3 = 70-89% effect and 4 = 90-100% effect. In the table below, the following letters are used to denote the plant species:

a - Polygonum lapathifolium (Pale persicaria)
b - Galium aparine (cleavers)
c - Chrysanthemum segetum (corn marigold)
d - Alopecurus myosuroides(blackgrass)
e - Elymus repens (Couchgrass)
f - Avena fatua (wild oat)
g - Abutilon theophrasti (velvetleaf)
h - Cyperus rotundus (purple nutsedge)
i - Pharbitis purpurea (morningglory)
j - Echinochloa crus-galli (barnyardgrass)
k - Setaria viridis (green foxtail)
l - Solanum nigrum (black nightshade)

The results obtained were as follows:

| Ex | Kg/ha | a | b | c | d | e | f | g | h | i | j | k | l |
|---|---|---|---|---|---|---|---|---|---|---|---|---|---|
| 1 | 2.5 | 4 | 4 | 4 | 3 | 2 | 2 | 4 | | 3 | 2 | 2 | 3 |
| 2 | 2.5 | 4 | 4 | 4 | 3 | 0 | 0 | 4 | 4 | 4 | 4 | 4 | 4 |
| 3 | 2.5 | 4 | 4 | 4 | 4 | 4 | 2 | 4 | 4 | 4 | 4 | 4 | 4 |
| 4 | 1.0 | | 4 | 4 | 4 | 4 | 2 | 4 | 4 | 4 | 3 | 4 | 4 |
| 5 | 1.0 | | 4 | 4 | 1 | 0 | 1 | 4 | 4 | 3 | 2 | 2 | 3 |
| 6 | 1.0 | 4 | 4 | 4 | 3 | 2 | 1 | 4 | 4 | 4 | 3 | 3 | 4 |
| 7 | 1.0 | 3 | 3 | 2 | 2 | 2 | 2 | 2 | 0 | 0 | 2 | 2 | 4 |
| 8 | 1.0 | 4 | 4 | 4 | 3 | 3 | 0 | 4 | 4 | 3 | 3 | 3 | |
| 10 | 1.0 | 2 | 3 | 4 | 2 | 0 | 0 | 2 | 0 | 1 | 0 | 0 | 3 |
| 11 | 1.0 | 2 | 4 | 2 | 0 | 0 | 0 | 2 | 0 | 1 | 2 | 0 | 4 |
| 12 | 1.0 | 2 | 4 | 3 | 0 | 0 | 2 | 2 | 0 | 0 | 0 | 0 | 4 |
| 13 | 1.0 | 4 | 4 | 4 | 2 | 2 | 1 | 4 | 0 | 3 | 2 | 1 | 4 |
| 14 | 1.0 | 4 | 4 | 4 | 3 | 3 | 3 | 4 | 4 | 4 | 2 | 2 | |
| 15 | 1.0 | 4 | 4 | 4 | 3 | 3 | 0 | 4 | | 4 | 4 | 4 | |
| 16 | 1.0 | 3 | 4 | 4 | 2 | 2 | 0 | 3 | 0 | 2 | 0 | 0 | 4 |
| 17 | 1.0 | 2 | 3 | 4 | 2 | 2 | 0 | 2 | 0 | 0 | 0 | 0 | |

| Ex | Kg/ha | a | b | c | d | e | f | g | h | i | j | k | l |
|---|---|---|---|---|---|---|---|---|---|---|---|---|---|
| 18 | 1.0 | 4 | 3 | 2 | 2 | 0 | 0 | 0 | 0 | 0 | 0 | 0 | |
| 22 | 1.0 | 4 | 4 | 4 | 2 | 0 | 0 | 2 | 0 | 0 | 0 | 4 | |
| 23 | 1.0 | 4 | 3 | 2 | 0 | 0 | 0 | 2 | 0 | 0 | 0 | 2 | |
| 24 | 1.0 | 4 | 4 | 4 | 0 | 2 | 0 | 0 | 3 | 0 | 2 | 3 | 3 |
| 25 | 0.5 | 3 | 3 | 4 | 0 | 0 | 0 | 1 | 0 | 0 | 0 | 0 | 2 |
| 29 | 0.5 | 3 | 4 | 4 | 2 | 2 | 2 | 4 | 4 | 4 | 2 | 0 | 4 |
| 30 | 0.5 | 2 | 4 | 2 | 2 | 2 | 0 | 2 | 0 | 3 | 0 | 0 | 3 |
| 31 | 0.5 | 2 | 4 | 4 | 2 | 2 | 0 | 4 | 4 | 4 | 3 | 2 | 4 |
| 40 | 1.0 | 3 | 4 | 4 | 0 | 0 | 0 | 2 | 2 | 0 | 0 | 0 | 4 |
| 41 | 0.5 | 4 | 4 | 4 | 2 | 0 | 0 | 4 | 4 | 2 | 3 | 0 | 4 |
| B1 | 1.0 | 4 | 4 | 4 | 4 | 3 | 2 | 4 | 4 | 4 | 3 | 4 | |
| B2 | 1.0 | 4 | 4 | 4 | 0 | 0 | 4 | 3 | 4 | 2 | 4 | 3 | 0 |
| B3 | 1.0 | 4 | 4 | 4 | 2 | 0 | 0 | 0 | 0 | 2 | 0 | 0 | |
| B5 | 0.5 | 4 | 4 | 4 | 3 | 0 | 3 | 2 | 0 | 0 | 2 | 4 | 4 |
| B6 | 0.5 | 4 | 4 | 4 | 2 | 3 | 0 | 2 | 4 | 2 | 2 | 0 | 4 |
| B7 | 0.5 | 4 | 4 | 4 | 2 | 2 | 2 | 0 | 3 | 0 | 2 | 2 | 4 |

HERBICIDAL EXAMPLE B (Post-Emergence)

Seeds of the plant species listed below were sown in anodised aluminium pans, 19 cm long x 9.5 cm x 6 cm deep containing sterilised sandy loam. They were then watered and placed in a controlled environment room (20°C; 75-95% relative humidity; 14 hours per day artificial illumination). Fourteen or twenty one days after sowing (depending on the species but when most plants had 2 to 3 true leaves) the seedlings received a foliar spray of the compounds of the Examples listed below, formulated as a solution/suspension in 1:1 by volume of acetone and the wetting agent polyoxyethylene (20 mols) monolaurate solution (2 g per litre).

The concentration of each test compound was calculated to give the desired rate of application of the compound in 450 litres per hectare. After 2 to 3 weeks growth in the controlled environment room the plants were visually assessed for any herbicidal response.

All differences from an untreated control were scored according to an index where 0 = no effect, 1 = 1-24% effect, 2 = 25-69% effect, 3 = 70-89% effect and 4 = 90-100% effect. In the table below, the following letters are used to denote the plant species:

a - Polygonum lapathifolium (Pale persicaria)
b - Galium aparine (cleavers)
c - Chrysanthemum segetum (corn marigold)
d - Alopecurus myosuroides (blackgrass)
e - Elymus repens (Couchgrass)
f - Avena fatua (wild oat)

g - Abutilon theophrasti (velvetleaf)
h - Cyperus rotundus (purple nutsedge)
i - Pharbitis purpurea (morningglory)
j - Echinochloa crus-galli (barnyardgrass)
k - Setaria viridis (green foxtail)
l - Solanum nigrum (black nightshade)

The results obtained were as follows:

| Ex | Kg/ha | a | b | c | d | e | f | g | h | i | j | k | l |
|---|---|---|---|---|---|---|---|---|---|---|---|---|---|
| 1 | 2.5 | 4 | 3 | 4 | 2 | 0 | 0 | 1 | | 1 | 1 | 2 | 4 |
| 2 | 2.5 | 4 | 4 | 4 | 3 | 1 | 2 | 4 | 3 | 4 | 4 | 3 | 4 |
| 3 | 2.5 | 3 | 4 | 3 | 3 | 2 | 2 | 4 | 2 | 3 | 4 | 4 | 3 |
| 4 | 1.0 | 3 | 3 | 3 | 3 | 2 | 2 | 4 | 1 | 2 | 2 | 2 | 3 |
| 5 | 1.0 | 3 | 3 | 2 | 0 | 0 | 1 | 1 | 0 | 1 | 1 | 2 | 0 |
| 6 | 1.0 | 4 | 3 | 2 | 2 | 0 | 1 | 1 | 1 | 1 | 1 | 1 | 2 |
| 8 | 1.0 | | 4 | 3 | 2 | 2 | 1 | 4 | 1 | 2 | 1 | 1 | 3 |
| 10 | 1.0 | 1 | 4 | 2 | 0 | 0 | 0 | 1 | 1 | 1 | 1 | 1 | 2 |
| 12 | 1.0 | 2 | 4 | 3 | 0 | 0 | 2 | 2 | 0 | 0 | 0 | 0 | 4 |
| 13 | 1.0 | 1 | 4 | 4 | 2 | 0 | 2 | 2 | 0 | 1 | 1 | 2 | 2 |
| 14 | 1.0 | 2 | 4 | 3 | 2 | 1 | 2 | 3 | 0 | 2 | 2 | 2 | 2 |
| 15 | 1.0 | 3 | 4 | 4 | 2 | 0 | 2 | 4 | 0 | 2 | 2 | 2 | 2 |
| 16 | 1.0 | 2 | 3 | 3 | 2 | 0 | 0 | 4 | 0 | 2 | 1 | 0 | 2 |
| 22 | 0.5 | 3 | 4 | 3 | 0 | 0 | 0 | 0 | 0 | 1 | 0 | 0 | 0 |
| 24 | 1.0 | 4 | 4 | 4 | 0 | 2 | 0 | 0 | 3 | 0 | 2 | 3 | 3 |
| 27 | 0.5 | 2 | 4 | 4 | 3 | 3 | 3 | 2 | 1 | 1 | 2 | 0 | 0 |
| 40 | 1.0 | 1 | 4 | 3 | 0 | 0 | 0 | 1 | 0 | 0 | 1 | 0 | 2 |
| 41 | 0.5 | 2 | 4 | 2 | 1 | 0 | 0 | 2 | 2 | 1 | 1 | 0 | 2 |
| B1 | 1.0 | 2 | 4 | 4 | 2 | 0 | 2 | 3 | 1 | 2 | 3 | 2 | 3 |
| B2 | 1.0 | 4 | 4 | 4 | 0 | 0 | 4 | 3 | 4 | 2 | 4 | 3 | 0 |
| B5 | 0.5 | 1 | 4 | 3 | 0 | 0 | 2 | 2 | 0 | 2 | 0 | 0 | 3 |

| | | | | | | | | | | | | | |
|---|---|---|---|---|---|---|---|---|---|---|---|---|---|
| B6 | 0.5 | 2 | 4 | 2 | 2 | 2 | 0 | 0 | 2 | 0 | 2 | 0 | 2 |
| B7 | 0.5 | 2 | 3 | 4 | 0 | 0 | 0 | 0 | 3 | 0 | 0 | 0 | 0 |

## Claims

1. The triazole sulphonamides of the formula:

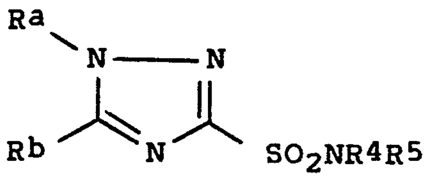

(I)

and the salts thereof, where $R^a$ represents hydrogen and $R^b$ represents a group $R^3CONHCR^2=CR^1$-, or $R^a$and $R^b$ together represent a chain of formula
$-C(R^3)=N-C(R^2)=C(R^1)$-or
$-C(=Y)-N(R^6)-C(R^2)=C(R^1)-$, where $R^1$, $R^2$ and $R^3$, which may be the same or different, each represent hydrogen, hydroxy, mercapto, halo, cyano, amino, a]kylamino, dialkylamino, or substituted or unsubstituted alkyl, aryl, aralkyl, alkoxy or alkylthio, Y is oxygen or sulphur, and $R^6$ is hydrogen or a substituted or unsubstituted alkyl or aryl group; or $R^1$ and $R^2$ together represent $-CH=CH-CH=CH$-or $-(CH_2)n$-where n is 3, 4 or 5; $R^4$ represents substituted phenyl or a substituted or unsubstituted aralkyl or heteroaryl group; and $R^5$ represents hydrogen, acyl, carboxy, alkoxycarbonyl, alkylsulphonyl, or a substituted or unsubstituted alkyl, alkenyl, alkynyl, aryl or aralkyl group, or a group of the formula:

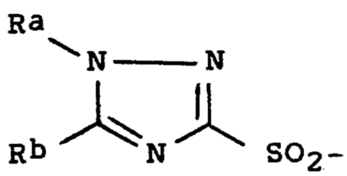

(A)

where $R^a$ and $R^b$ are as defined hereinbefore.

2. The triazole sulphonamides according to claim 1 wherein $R^1$ represents hydrogen, chloro, bromo, or alkyl of 1 to 6 carbon atoms optionally substituted by one or more halogen atoms or alkoxy groups of 1 to 4 carbon atoms.

3. The triazole sulphonamides according to claim 1 or claim 2 wherein $R^2$ represents hydrogen, phenyl, or alkyl of 1 to 6 carbon atoms optionally substituted by one or more halogen atoms or alkoxy groups of 1 to 4 carbon atoms.

4. The triazole sulphonamides according to any of claims 1 to 3 wherein $R^3$ represents hydrogen, phenyl, or alkyl of I to 6 carbon atoms optionally substituted by one or more halogen atoms or alkoxy groups of 1 to 4 carbon atoms.

5. The triazole sulphonamides according to any of claims 1 to 4 wherein $R^4$ represents a phenyl group substituted by one or more halogen atoms, alkyl groups of 1 to 4 carbon atoms, alkoxy or alkylthio groups of 1 to 4 carbon atoms, alkoxycarbonyl groups of 2 to 5 carbon atoms or nitro groups.

6. The triazole sulphonamides according to any of claims 1 to 5 wherein $R^5$ represents hydrogen, alkyl of 1 to 6 carbon atoms, alkenyl or alkynyl of 2 to 6 carbon atoms, or acyl, alkoxycarbonyl or alkylsulphonyl in which the alkyl moiety is of 1 to 4 carbon atoms.

7. The triazole sulphonamides according to any of claims 1 to 6 wherein $R^a$ and $R^b$ together represent a chain of formula $-C(R^3)=N-C(R^2)=C(R^1)$-, $R^1$, $R^2$ and $R^3$ each represent hydrogen, methyl, ethyl, phenyl, trifluoromethyl or chloro, $R^4$ is a phenyl group substituted by one or more halogen atoms, alkyl, alkoxy or alkylthio groups of 1 to 4 carbon atoms, trifluoromethyl groups, alkoxycarbonyl groups of 2 to 5 carbon atoms, or nitro groups, and $R^5$ is hydrogen.